# EUROPEAN PATENT APPLICATION

(11) **EP 0 987 042 A2**
(43) Date of publication of application: **22.03.2000**
(21) Application number: 99117447.5
(22) Date of filing: 08.09.1999
(51) Int. Cl.: A61M 25/00, A61M 25/098

(54) **Design and method to fabricate PTCA balloon radiopaque marker band**

(30) Priority: 15.09.1998 US 153878; 15.09.1998 US 153791; 15.09.1998 US 153815; 15.09.1998 US 153722; 15.09.1998 US 153623; 15.09.1998 US 153520; 15.09.1998 US 153880
(71) Applicant: Medtronic Inc., Minneapolis, MN 55432-3576 (US)
(72) Inventor: Lentz, David J., La Jolla, CA 92037 (US); Wu, Pin-Pin, San Diego, CA 92128 (US); Fortuney, Alain, San Diego, CA 92106 (US); Davison, Paul, Poway, CA 92064 (US); Fugoso, Mauricio, Chula Vista, CA 91910 (US); Brahana, Christopher T., San Diego, CA 92111 (US)
(74) Representative: Hughes, Andrea Michelle

(57) **Abstract**

A radiopaque marker for the body portion of a medical catheter, and the methods for manufacturing such a marker, require the blending of a metal with a polymer matrix. For the blend, the metal is preferably greater than approximately seventy percent by weight. This blend is then made into a band having a thickness that is less than about two thousandths of an inch. To assemble the catheter, the radiopaque band is positioned against the catheter body and the band is then thermally bonded to the catheter body. For a balloon catheter, the catheter body will be tubular and the radiopaque marker band can be formed as a ring that will surround the catheter body, circumscribe the lumen inside the catheter body, or be incorporated as part of the catheter body. The position of the marker band can be established as desired and then thermally bonded onto the catheter body to identify the location of the balloon on the catheter body.

## Description

The present invention pertains generally to medical catheters. More particularly, the present invention pertains to the manufacture and use of radiopaque marker bands which can be positioned on a medical catheter to identify the location of the catheter during surgical procedures using fluoroscopic techniques. The present invention is particularly, but not exclusively, useful as a polymer based radiopaque marker which is flexible, and which has a low profile that will facilitate the insertion of the catheter into a body cavity.

A well known technique for precisely controlling the location of a catheter as it is being positioned in a vessel of the cardio-vascular system of a patient during angioplasty surgery is to monitor the catheter insertion using radiographic equipment. Such a procedure, however, requires that certain components of the catheter be somehow identified with radiopaque markers. Typically, the radiopaque markers which have been used for this purpose are made entirely of metal. For example, U.S. Patent 4,793,359 which issued to Sharrow for an invention entitled "Centering Balloon Structure For Transluminal Angioplasty Catheter" discloses radiopaque markers made of platinum or gold. Alternatively, U.S. Patent 5,034,005 which issued to Appling for an invention entitled "Radiopaque Marker" discloses radiopaque markers made of a surgical grade stainless steel. Although effective for the intended purpose, metal markers have their shortcomings.

Metal markers, of course, are not flexible. Flexibility, however, is a very desirable quality for an angioplasty catheter as it is necessary for improved tracking of the catheter through the coronary tree of a patient. Further, metal markers typically have rough edges and burrs which can present problems if not smoothed. For example, it is known that burrs on metal radiopaque markers are capable of causing pinholes in the balloon of an angioplasty catheter. If detected early enough, the catheter is simply not used. Unfortunately, it sometimes happens that the adverse effects caused by burrs can go unnoticed until after the catheter has been placed in use. This presents many complications which can sometimes have serious consequences. Simply stated, such consequences should be avoided if at all possible.

In addition to the shortcomings noted above, metal markers can sometimes present a restrictive profile. Generally, it is true that the wall thickness of a metal marker is added directly to the outside diameter of the catheter as it is being advanced through a vessel. One way to mitigate this increase in profile is to embed the metal markers in the catheter shaft, as disclosed in U.S. Patent Application 08/977,733, filed by Fugoso and Rowean for an invention entitled "Imbedded Marker And Flexible Guide Wire Shaft" which is assigned to the same assignee as the present invention. Another method disclosed in U.S. Patent Application 09/046,241 filed by Rafiee and Squadrito for an invention entitled "Catheter Having Extruded Radiopaque Stripes Embedded In Soft Tip And Method Of Fabrication" which is assigned to the same assignee as the present invention is to co-extrude a "radiostripe" made of a mix of molten polymer and a radiopaque powder during extrusion of the distal tip. Finally, in addition to flexibility and profile concerns, it is well known that when a completely metallic marker is to be used, it is necessary to bond or attach the marker to the angioplasty catheter using an adhesive. In general, the tasks that are required to attach a metal marker to a catheter are labor intensive and costly.

In light of the above, it is an object of the present invention to provide a marker for a medical device which has improved flexibility for tracking through the vessels of a patient's cardio-vascular system. Another object of the present invention is to provide a marker having a wall thickness of only about 0.025 mm (one thousandth of an inch) in order to present a reduced profile for an angioplasty catheter and thereby improve the catheter's ability to traverse small vessels, and lesions or stents in the vessel. Still another object of the present invention is to provide a marker for a medical device which does not present burrs or rough edges which require additional smoothing. Yet another object of the present invention is to provide a marker for a medical device which does not require the use of adhesives when affixing the marker to the device. Another object of the present invention is to provide methods for manufacturing an angioplasty balloon with radiopaque markers which are simple and easy to accomplish. Still another object of the present invention is to provide a marker for a medical device which is relatively cost effective.

A radiopaque marker for a medical device, such as a balloon angioplasty catheter, includes a polymer matrix material which is blended with a metal. For the present invention the polymer matrix material is preferably a polyether block amide co-polymer, and the metal is preferably selected from the group which includes tungsten, silver, gold, platinum and their alloys. Further, the blend should include the metal at about seventy percent, or more, by weight. An effective blend for the present invention has been a mixture of ninety percent tungsten, by weight, and a polymer which is commercially available under the trade name of Pebax®.

In accordance with the methods of the present invention, the matrix material and metal blend is first formed as a band. In particular, for its use with a balloon angioplasty catheter, the radiopaque band is formed as a ring which is dimensioned to fit around a tubular shaped catheter body, circumscribe the lumen inside the catheter body, or be incorporated as part of the catheter body. Importantly, for the present invention, the marker band and the catheter body are made of the same polymer material, or of compatible polymer materials. In this sense, compatibility means that the materials should melt into each other. Regardless of which specific materials are used, once the band has been positioned as desired, it is thermally bonded to portions of the tubular catheter body using r.f. energy. To do this, an active RF mandrel is inserted through the lumen of the tubular shaped catheter body and an RF coil is positioned so that the catheter body and the marker band are located between the mandrel and the coil. A current is then applied to the coil to generate the RF energy that is needed to melt both the marker band and the catheter body. The result is an integral thermal bond between the marker band and the catheter body.

As intended for the present invention, a single marker band can be used, or a plurality of marker bands can be used and positioned anywhere on the tubular catheter body to landmark specific locations or components. Possible marker band positions include, the extreme distal tip of the catheter, the center of the balloon, brackets for stents, and guidewire entry or exit ports. Further, the radiopaque bands used for the markers need not be formed as rings. Instead, they can be formed as strips and affixed to the catheter body for similar purposes.

The novel features of this invention, as well as the invention itself, both as to its structure and its operation, will be best understood from the accompanying drawings, given by way of example only, and taken in conjunction with the accompanying description, in which similar reference characters refer to similar parts, and in which:
Fig. 1 is a perspective view of the balloon catheter of the present invention with the component subassemblies interconnected with each other;
Fig. 2 is an exploded perspective view of the catheter of the present invention showing the interconnective relationships between the component subassemblies;
Fig. 3A is a cross sectional view of the balloon subassembly of the present invention as seen along the line 3-3 in Fig. 1;
Fig. 3B is a side elevational view of the guidewire marker tube illustrating an alternative method for its assembly;
Fig. 4 is a side elevational view of the distal portion of the balloon positioned over the distal end of the guidewire-marker tube with portions broken away and shown in phantom for clarity;
Fig. 5 is an elevation cross sectional view of the balloon and guidewire-marker tube as shown in Fig. 4 and positioned in a heating die for thermally bonding the balloon to the distal end of the guidewire-marker tube;
Fig. 6 is a side elevational view of the distal tip of the catheter which results from the bonding process depicted in Fig. 5;
Fig. 7 is a perspective view of the coupling tube in a position to be joined with the proximal portion of the balloon and the distal end of dual lumen tube;
Fig. 8 is a cross sectional view of the coupling tube after it has been joined with the proximal portion of the balloon and the distal end of the dual lumen tube as would be seen along the line 8-8 in Fig. 7;
Fig. 9 is a cross sectional view of the coupling tube and guidewire marker tube as would be seen along the line 9-9 in Fig. 8 after the coupling tube has been joined with the distal end of the dual lumen tube;
Fig. 10 is a perspective view of the proximal portion of the balloon positioned for interconnection with both the proximal end of the guidewire-marker tube, and with the distal end of the dual lumen tube;
Fig. 11 is a cross sectional view of the components shown in Fig. 10 when positioned for integral bonding with each other in accordance with an alternative embodiment of the catheter of the present invention;
Fig. 12 is a cross sectional view of the catheter portion between the balloon assembly and the mid-section which results from the bonding process depicted in Fig. 11;
Fig. 13 is a cross sectional view of the dual lumen tube of the present invention as would be seen along the line 13-13 in Fig. 12;
Fig. 14 is a cross sectional view of the transition "necked-down" region between the dual lumen tube and the balloon assembly as would be seen along the line 14-14 in Fig. 12;
Fig. 15A is a perspective view of the distal end of the hypotube positioned for insertion into the proximal end of the mid-tube for interconnection therewith, and the distal end of the mid-tube for interconnection with the proximal end of the dual lumen tube;
Fig. 15B is a cross sectional view of the hypotube as seen along the line 15-15 in Fig. 15A;
Fig. 16 is a cross sectional view of the distal end of the hypotube inserted for bonding with the mid-tube as would be seen along the line 16-16 in Fig. 15A;
Fig. 17 is a cross sectional view of the interconnection between the hypotube, mid-tube, and the dual lumen tube which results from the bonding process depicted in Fig. 16;
Fig. 18 is a perspective view of the catheter of the present invention shown in operative association with a guidewire and an inflator;
Fig. 19 is a schematic of the components used in the method of laminating a hypotube subassembly for use within the medical catheter of the present invention; and
Fig. 20 is a cross sectional view of the die of the extruder depicted as would be seen along the line 20-20 in Fig. 19.

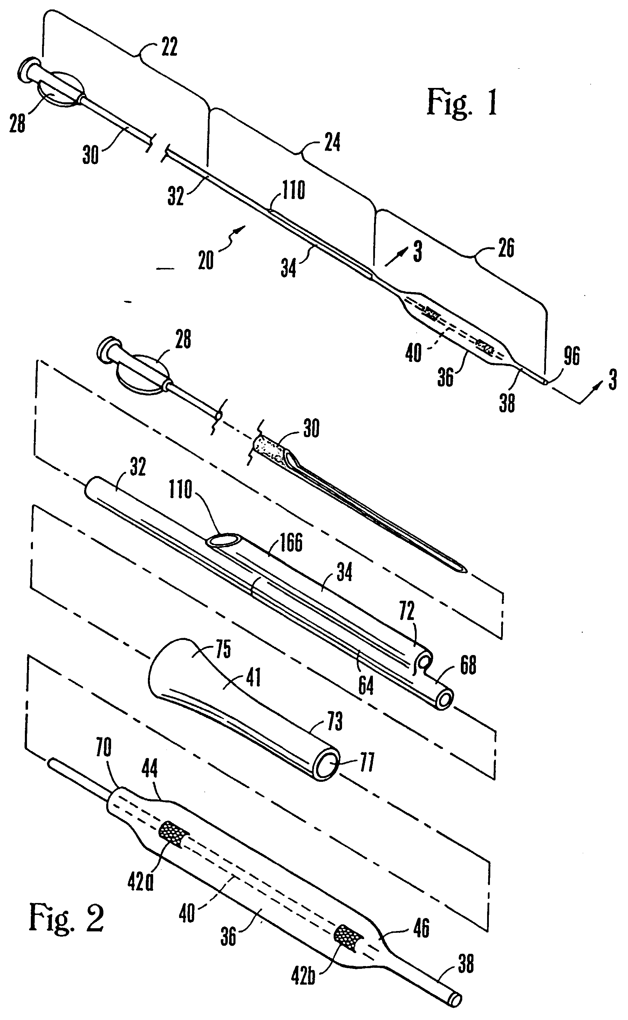

Referring initially to Fig. 1, a percutaneous transluminal coronary angioplasty (PTCA) catheter which has been manufactured in accordance with the methods of the present invention is shown and generally designated 20. In overview, the catheter 20 includes three separate and distinct subassemblies. In Fig. 1, these subassemblies are shown in their proximal-to-distal order as: a hypotube subassembly 22, a mid-section subassembly 24, and a balloon subassembly 26. As intended for the present invention, and disclosed herein, each of the subassemblies 22, 24 and 26, can be individually fabricated in separate manufacturing operations. The various subassemblies 22, 24 and 26 can then be subsequently joined together to create the catheter 20.

For the general dimensions that are presented in the final assembly of the catheter 20, it is to be appreciated that the overall length of the catheter 20 is preferably in the range of about 1350 mm ± 30 mm. Of this overall length, the hypotube subassembly 22 will be approximately one thousand and twenty millimeters (1020 mm), the mid-section subassembly 24 will be approximately three hundred millimeters (300 mm), and the balloon subassembly 26 will be approximately thirty millimeters (30 mm). However, due to the flexibility which is afforded by the separate subassembly manufacturing operations, the final product can include subassemblies 22, 24 and 26 which have been specially tailored and sized for the specific operational requirements of the catheter 20. Further, as an additional advantage of this flexibility, when a defect is detected in a pre-assembled subassembly 22, 24 or 26, only the defective subassembly needs to be discarded. Such a defect does not thereby result in a loss of the entire catheter 20.

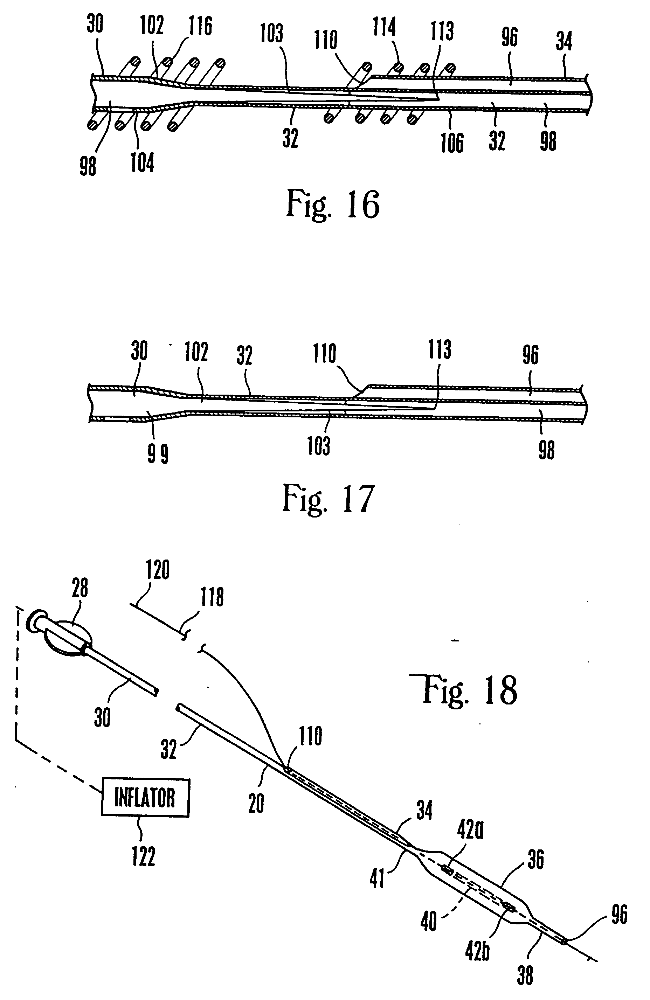

As shown in Fig. 1, the hypotube subassembly 22 includes a luer fitting 28 and a hypotube 30 which extends distally from the luer fitting 28. For purposes of the present invention, the luer fitting 28 can be of any type well known in the pertinent art. Further, it can be made of a material that is well known in the pertinent art, such as a medical grade plastic. The hypotube 30 includes a hollow core tube which is preferably made of a stainless steel which is laminated with an external coating of a polymer. A suitable polymer for this purpose is a polyether block amide co-polymer, such as manufactured by Elf Atochem Corporation, and commercially available under the trademark Pebax®. Specifically, a suitable material for use as the laminate on hypotube 30 is Pebax® 7033. Further, the laminated polymer coating over the core tube is preferably colored blue for the purpose of visually contrasting the hypotube 30 from other components of the catheter 20. As also shown in Fig. 1, the mid-section subassembly 24 includes a mid-tube 32 which is joined to a dual lumen tube 34. In the mid-section subassembly 24, the mid-tube 32 is preferably made of a blue colored block co-polymer, such as Pebax® 7223. On the other hand, the dual lumen tube 34 is preferably made of a material which consists of about ninety percent (90%) polymer, and ten percent (10%) graphite. In this combination, Pebax 7233 is a suitable material for the polymer. This combination of polymer and graphite as used for the dual lumen tube 34 has two significant operational aspects. Firstly, the combination makes the dual lumen tube 34 black in color. Thus, for operational purposes, it is easily distinguished from other parts of the catheter 20 which are primarily blue in color. This then allows the operator to more easily identify the location of the guidewire port 110 (see Fig. 18) which will be substantially located at the margin between the mid-tube 32 and the dual lumen tube 34. Secondly, the graphite in the combination gives the dual lumen tube 34 enhanced lubricity to facilitate insertion and passage of a guidewire 118 (see Fig. 18) through the dual lumen tube 34.

The balloon subassembly 26 is shown in Fig. 1 to include a balloon 36 and a distal tip 38. Also, in phantom, it can be seen that the balloon subassembly 26 includes a guidewire-marker tube 40 which is located inside the balloon 36 and extends longitudinally along the length of the balloon 36. For the present invention, the balloon 36 is preferably made of Pebax® 7033, while the guidewire-marker tube 40 is preferably made of a blue colored Pebax® 7233. For purposes of the present invention, the balloon 36 can be manufactured in accordance with the disclosure set forth in U.S. Application Serial No. 09/002,676 for an invention entitled "Method for Making a Medical Balloon Catheter" which was filed on January 5, 1998,and which is assigned to the same assignee as the present invention. As more fully set forth below, the distal tip 38 results from the mixed melting of the balloon 36 and the guidewire-marker tube 40. Thus, for the example provided above, the distal tip 38 includes both the Pebax 7033 and Pebax 7233 polymer material.

It is to be noted at this point that the preferred polymer materials which have been selected for the fabrication of the various subassemblies 22, 24 and 26 of the catheter 20 are compatible with each other. Specifically, the selected polymer materials are compatible with each other in the sense that the Pebax® 7033, which is the preferred material used for the hypotube 30 and the balloon 36, is capable of being thermally bonded with the Pebax 7233, which is the preferred material used for the mid-tube 32, the dual lumen tube 34, the coupling tube 41, and the guidewire-marker tube 40. This is important because, as illustrated in Fig. 2, the hypotube 30 of the hypotube subassembly 22 is to be thermally bonded to the mid-tube 32 of the mid-section subassembly 24. Also, the coupling tube 41 is to be thermally bonded to the dual lumen tube 34 of the mid-section subassembly 24 and to the balloon 36 of the balloon subassembly 26. Additionally, thermal or heat bonding between the various parts of the subassemblies 22, 24 and 26 is also intended for the present invention. The skilled artisan, however, will appreciate that adhesives can be used as an alternative to thermal bonding, if appropriate.

It is to be understood that materials other than the preferred polymer materials (i.e. Pebax®) disclosed herein can be used for the assembly and manufacture of the catheter 20. For example, a Nylon 12 material that is commercially available under the trademark "VESTAMID" or a polyurethane may be suitable for the present invention. Indeed, the particular material to be used is a matter of design choice which is primarily dependent on the particular characteristics desired for the balloon. For purposes of comparison, it is to be noted that the competing characteristics for materials that are to be used for the manufacture of the catheter 20 involve a trade-off between stiffness, for column strength in the catheter body, and softness, for flexibility. On the one hand, the catheter should not be too stiff, because with increased stiffness there is also an increased susceptibility for the catheter to "kink." On the other hand, if the material is too soft, it will exhibit decreased "pushability." As a measure of the hardness of a material, durometer ratings are helpful. Larger or higher durometer readings indicate harder materials. For the materials suggested here the respective durometer ratings are: Nylon 12, 72-75 D; Pebax®, 65-72 D; and Polyurethane, 55-70 D.

It is important for the present invention that the particular polymer materials to be used will either be all the same polymer, similar polymers, or be polymers which are thermally compatible with each other. As used in the context of the present invention, thermal compatibility describes a condition wherein two mated polymer materials will bond together with no discernible interface when they are heated, i.e. they are miscible. Polymers which are identical are thermally compatible. Polymers, however, do not have to be identical to be thermally compatible.

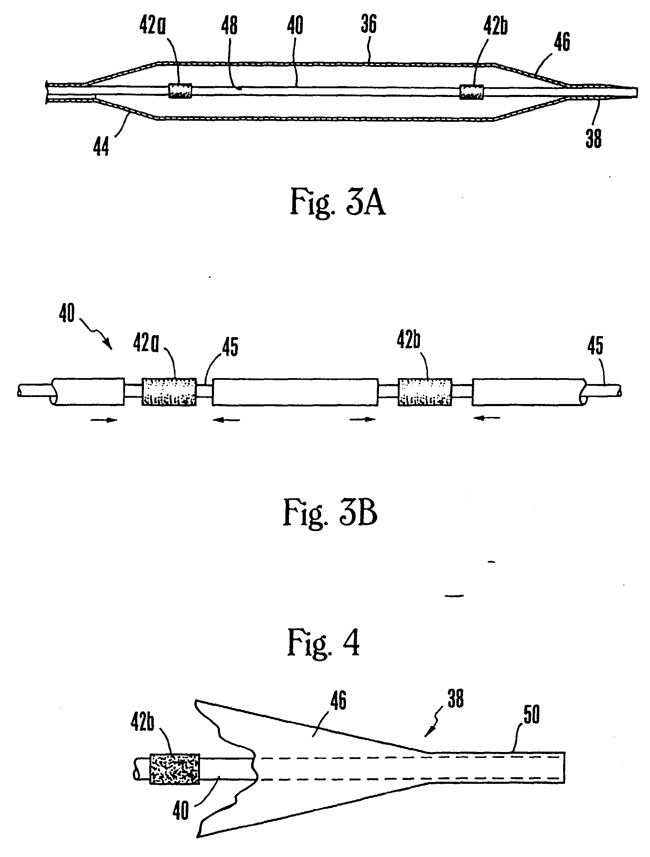

The balloon subassembly 26 of the catheter 20 is perhaps best appreciated by cross-referencing Fig. 2 with Figs. 3A and 3B. Of particular importance in the manufacture of the balloon subassembly 26 is the presence of marker bands 42a and 42b which have been bonded with or onto the guidewire-marker tube 40. In accordance with the present invention, each of the marker bands 42a,b is formed as a ring or tube before it is bonded onto the guidewire-marker tube 40.

The range of dimensions for the marker bands 42 is important for an appreciation of how they help in providing a reduced profile for the completely assembled catheter 20. Specifically, the marker bands 42a,b can have an inner diameter which is in the range of approximately 0.575 to 0.625 mm (twenty three to twenty five thousandths of an inch) (0.023 - 0.025 inch). For any particular diameter, the wall thickness of the marker bands 42a,b can be approximately 0.05 mm (two thousandths of an inch) (0.0020 inch). Further, they will have a length which is in the range of one to two millimeters (1 - 2 mm).

As intended for the present invention, the marker bands 42a,b are preferably made of a blend of metal and polymer materials. Specifically, a Pebax® polymer, such as Pebax® 5233, can be blended with a metal selected from the group including tungsten, silver, gold, platinum, or any of the other known radiopaque metals, and their alloys. Further, in this blend, the radiopaque metal should constitute greater than approximately seventy percent of the material by weight (> 70% wt.). Preferably, for the present invention, the marker bands 42a,b include, by weight, approximately ninety percent tungsten (90% W) and approximately ten percent polymer (10% Pebax®).

Figs. 2 and 3A show that the marker bands 42a and 42b are located on the guidewire-marker tube 40 inside the balloon 36. These Figs. also show that the marker bands 42a and 42b are located, respectively, under the proximal end 44 and the distal end 46 of the balloon 36. It is to be appreciated, however, that several variations in both the location and length of the marker bands 42a,b are possible. For example, marker bands 42 can be positioned underneath the balloon 36 to landmark either the center of the balloon 36, the entire length of the balloon 36, or the lengths of various stents (not shown) which may be used with the balloon 36. Additionally, other parts of the catheter 20 can be landmarked with marker bands 42, as desired. Regardless where the marker bands 42 are to be positioned on the catheter 20, it is to be appreciated that when a compatible polymer is used for their manufacture (e.g. Pebax ), the marker bands 42 can be thermally bonded to other polymer parts of the catheter 20 according to the desires of the manufacturer.

In accordance with the present invention, heat bonding of the marker bands 42 to the guidewire-marker tube 40 can be accomplished in any manner well known in the pertinent art. For example, radio frequency (RF) energy can be used to generate temperatures of around 177°C (three hundred and fifty degrees Fahrenheit) (350 F) to thermally bond the marker bands 42 to the guidewire-marker tube 40. Further, although Figs. 2 and 3A indicate that the marker bands 42a,b are positioned around the outside surface of the guidewire-marker tube 40, it is to be understood that the marker bands 42a,b could just as easily be bonded to the inside surface of the marker tube 40. In this case, the marker bands 42a,b would be inside the lumen 48 of the guidewire-marker tube 40. Alternatively, as shown in Fig. 3B, the marker bands 42a,b can be butt joint bonded into the marker tube 40. For all configurations of the marker bands 42 on tube 40, an active mandrel 45 can be used with the marker bands 42 and sections of tube 40 arranged as desired. For purposes of the present invention, active mandrels are taken to be ferrous (Fe) based materials which interact with RF energy to generate heat. This is in contrast with inactive mandrels which are made of inert materials such as gold (Au) or copper (Cu). Further, for the present invention it is preferable to use Teflon coated mandrels which will facilitate removal of the polymer from the mandrel after the bonding operation. As will be appreciated by the skilled artisan, the dimensions and shape of a particular mandrel will determine the configuration that is taken by the polymer as it melts. This, in turn, establishes the subsequently permanent configuration of the polymer after it is cooled and removed from the mandrel.

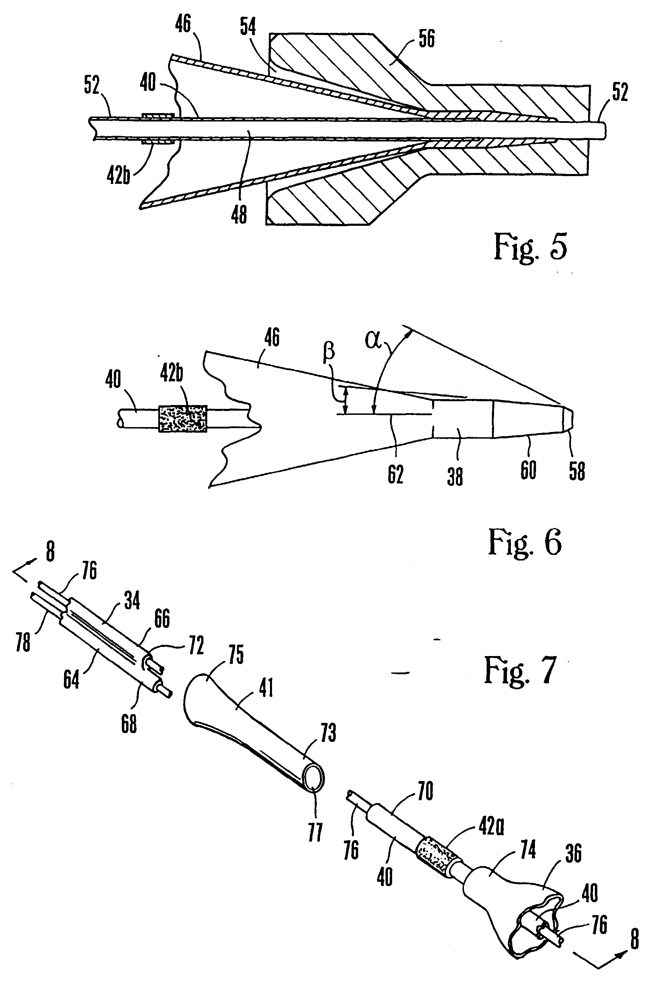

During the manufacture of the distal tip 38, the distal tail 50, which extends from the distal end 46 of balloon 36, is positioned over the guidewire-marker tube 40 substantially as shown in Fig. 4. As shown in Fig. 5, an active radio frequency (RF) mandrel 52 is then passed through the lumen 48 of the guidewire-marker tube 40. This combination is then inserted into the cavity 54 of a mold 56. There, it is heated to a temperature in a range of approximately 99 top 177°C (two hundred and ten to three hundred and fifty degrees Fahrenheit) (210 F - 350 F). This operation melts both the Pebax material of the balloon 36 and the Pebax material of the guidewire-marker tube 40 in the mold 56. This simultaneous melting provides for an integral bonding between the balloon 36 and the guidewire-marker tube 40 in the vicinity of the distal tip 38.

Fig. 6 shows the result of the above described operation. Specifically, the resultant distal tip 38 is preferably formed by the mold 56 to have both an end taper 58 and a transition taper 60. The end taper 58, indicated by the angle α in Fig. 6, provides for a region of increasing outside diameter in the proximal direction for the distal tip 38. The angle α which is definitive of the end taper 58 is measured from the centerline 62, and can generally be in the range of about fifteen to about seventy five degrees (15 - 75 ). Preferably, the angle α will be around sixty degrees (α = 60 ). The transition taper 60, indicated by the angle β in Fig. 6, provides for another region of increasing outside diameter for the distal tip 38. This increase is also in the proximal direction. For the transition taper 60 the angle β, which is also measured from the centerline 62, will generally be in the range of about four to about six degrees (4 - 165°). Preferably the angle β will be around five degrees (β = 5 ).

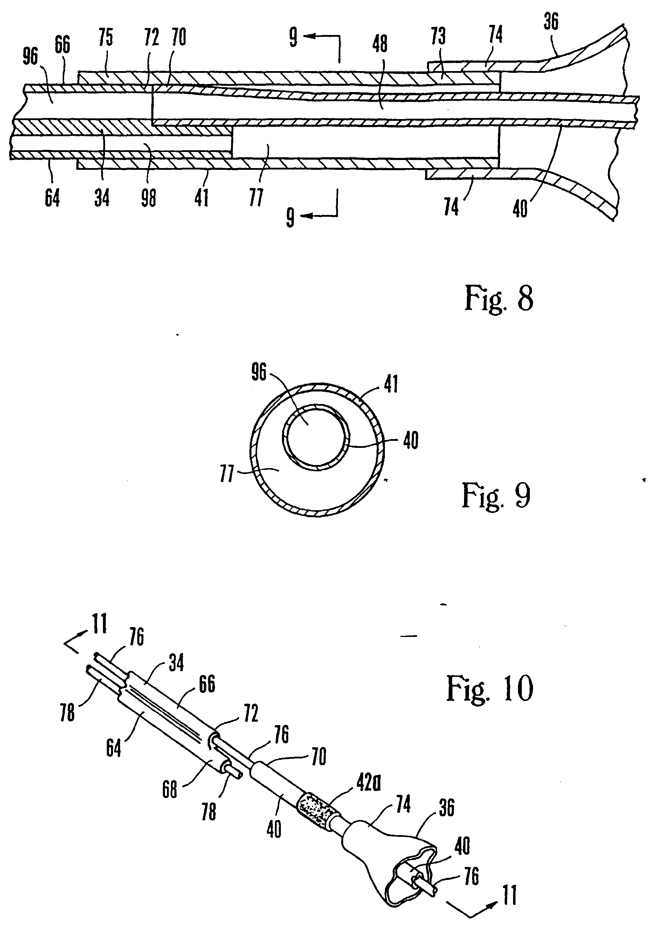

In the assembly of the catheter 20, the midsection subassembly 24 can be connected with the balloon subassembly 26 in either of two ways. One way uses a coupling tube 41 (see Figs. 7-9). The other way does not (see Figs. 10-12). In either case, several parts of the catheter 20 need to be uniquely configured. In particular, for this purpose it will be noted in Figs. 2, 7 and 10 that the dual lumen tube 34 is formed with an inflation section 64 and a guidewire section 66. Specifically, the dual lumen tube 34 can be extruded in a manner well known in the pertinent art. Extrusion alone, however, does not suffice for the fabrication of the dual lumen tube 34. As shown in Figure 7, the inflation section 64 includes an extension 68 which projects beyond the guidewire section 66 through a distance of approximately two millimeters. Using this configuration for dual lumen tube 34, it can be seen that the proximal end 70 of the guidewire-marker tube 40 can be positioned to abut the distal end 72 of the guidewire section 66 of the dual lumen tube 34. This positioning of the guidewire-marker tube 40 against the distal end 72 of dual lumen tube 34 also brings the outer surface of the proximal end 70 of guidewire-marker tube 40 into contact with the outer surface of the extension 68 of dual lumen tube 34.

For one embodiment of the present invention, a coupling tube 41, which is approximately five centimeters (5cm) in length, is incorporated into the catheter 20. One reason this is done is to establish a greater separation between those points on the catheter 20 which react to forces created by an inflation of the balloon 36. This increased separation may be necessary because it happens that as the balloon 36 is inflated, it generates forces on the catheter 20 which tend to elongate the catheter 20. A permanent set may result from this elongation which will cause a distortion of the balloon assembly 26 after deflation of the balloon 36. Such a distortion can easily inhibit withdrawal of the catheter 20 from the patient, and should be avoided. By attaching the coupling tube 41 to one end of the balloon 36, however, the distance between points on the catheter 20 where an inflation of the balloon 36 exerts stretching forces can be effectively increased. Specifically, this increase will be by about the length of the coupling tube 41. The structure for this embodiment will be best appreciated with reference to Figs. 7 and 8.

In Fig. 7 it will be seen that the coupling tube 41 has a distal end 73 and a proximal end 75, with a lumen 77 extending through the coupling tube 41 between the ends 73, 75. Further, it is to be seen that the proximal end 75 of coupling tube 41 is flared to give the lumen 77 a larger inside diameter at the proximal end 75. This flaring can be accomplished in any manner well known in the pertinent art, such as by inserting an awl (not shown) into the lumen 77 at proximal end 75. By cross referencing Figs. 7 and 8 it will be appreciated that, in order to join the coupling tube 41 to the balloon 36, the proximal tail 74 of balloon 36 is positioned in a surrounding relationship over the distal end 73 of coupling tube 41. The proximal tail 74 is then thermally bonded to the distal end 73. To bond the coupling tube 41 to the mid-section assembly 24, the flared proximal end 75 of coupling tube 41 is positioned in a surrounding relationship over the distal end 72 of the dual lumen tube 34. Recall, the proximal end 70 of guidewire marker tube 40 also abuts the guidewire section 66 at the distal end 72 of dual lumen tube 34. The proximal end 75 of coupling tube 41 is then thermally bonded to the distal end 72 of the dual lumen tube 34. As best seen in Fig. 8, the connecting point between the proximal end 70 of guidewire marker tube 40 and the balloon 36 is effectively moved away from the balloon 36 by about the length of the coupling tube 41. As discussed above, this provides additional separation of about five centimeters (5 cm) between those points on the catheter 20 which react to the forces that are generated by an inflated balloon 36. For reasons discussed above, this is beneficial. There is, however, another benefit.

For the configuration of catheter 20 which incorporates the coupling tube 41, it can be an added benefit that the guidewire marker tube 40 is "coaxial" with the coupling tube 41. With this "coaxial" structure the flexibility of the catheter 20 is improved without degradation of the ability of catheter 20 to inflate the balloon 36. By referencing Fig. 9 it is to be appreciated that the guidewire marker tube 40 actually extends through the lumen 77 of coupling tube 41. With this relationship, the guidewire lumen 96 of dual lumen tube 34 is effectively continued through the guidewire marker tube 40. At the same time, the inflation lumen 98 of dual lumen tube 34 is placed in fluid communication with the lumen 77 of coupling tube 41, and with the interior of the balloon 36.

It is to be appreciated that the thermal bonding which is established between coupling tube 41 and the respective components of mid-section assembly 24 and balloon assembly 26 is accomplished using methods well known in the pertinent art. Essentially, these methods involve the use of active mandrels which hold and shape the polymer materials as they are heated by RF energy. As indicated throughout this disclosure, the position of mandrels, the dimensions and shape of the mandrels, and the location and activation of RF heating coils are a matter of design choice and are dictated by the desired results.

For another embodiment of the catheter 20, the connection between the mid-section assembly 24 and the balloon assembly 26 can be made without the use of a coupling tube 41 (see Figs. 10-12). With specific reference to Fig. 10, it will be appreciated that guidewire-marker tube 40 can be positioned against the dual lumen tube 34 as indicated above. Further, it is to be appreciated that the proximal tail 74 of balloon 36 can be moved into a position to surround both the distal end 72 and the extension 68 of the dual lumen tube 34. Simultaneously, the proximal tail 74 can be positioned over the proximal end 70 of guidewire-marker tube 40. With the balloon 36, guidewire-marker tube 40 and dual lumen tube 34 positioned as suggested, an active RF mandrel 76 can be sequentially inserted into the respective lumens of guidewire-marker tube 40 and the guidewire section 66 of dual lumen tube 34. At the same time, an active RF mandrel 78 can be inserted through the lumen of inflation section 64 of the dual lumen tube 34. This mandrel 78 is "active" in the sense that it heats up when exposed to RF energy. This combination is now ready for heat bonding.

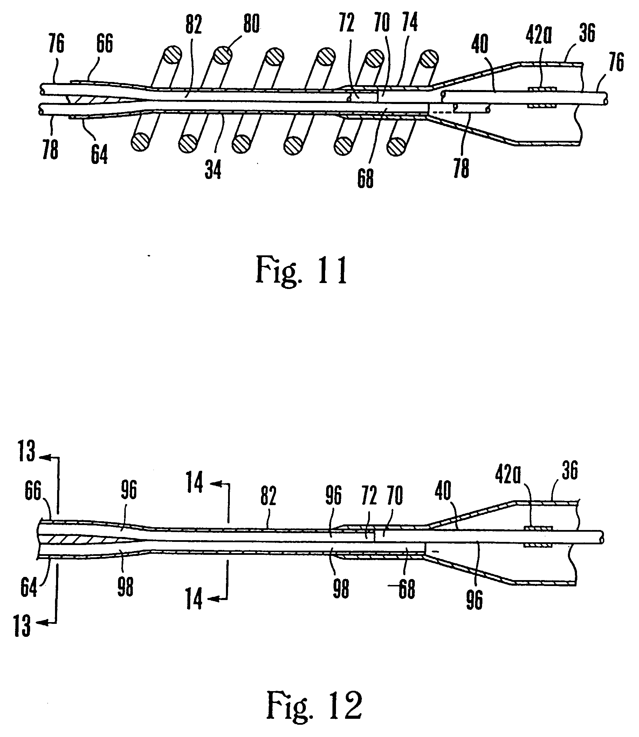

To effect a bonding between the proximal tail 74 of the balloon 36, the guidewire-marker tube 40, and the dual lumen tube 34, a radio frequency (RF) coil 80 can be positioned, substantially as shown in Fig. 11. Upon activation of the coil 80, these components will bond wherever they are in contact with each other. Activation of the coil 80 actually serves two purposes. Firstly, there is the bonding action just described. Secondly, the coil 80 can be extended over at least a portion of the dual lumen tube 34 to create a "necked-down" portion 82 of the dual lumen tube 34. As best seen in Fig. 11, this necked-down portion 82 will be immediately proximal to the balloon subassembly 26 and, preferably, will be approximately four centimeters in length (4 cm.). Upon deactivation of the coil 80, the coil 80 and the two mandrels 76 and 78 are removed. The result of this bonding and "necking-down" operation is shown in Fig. 12.

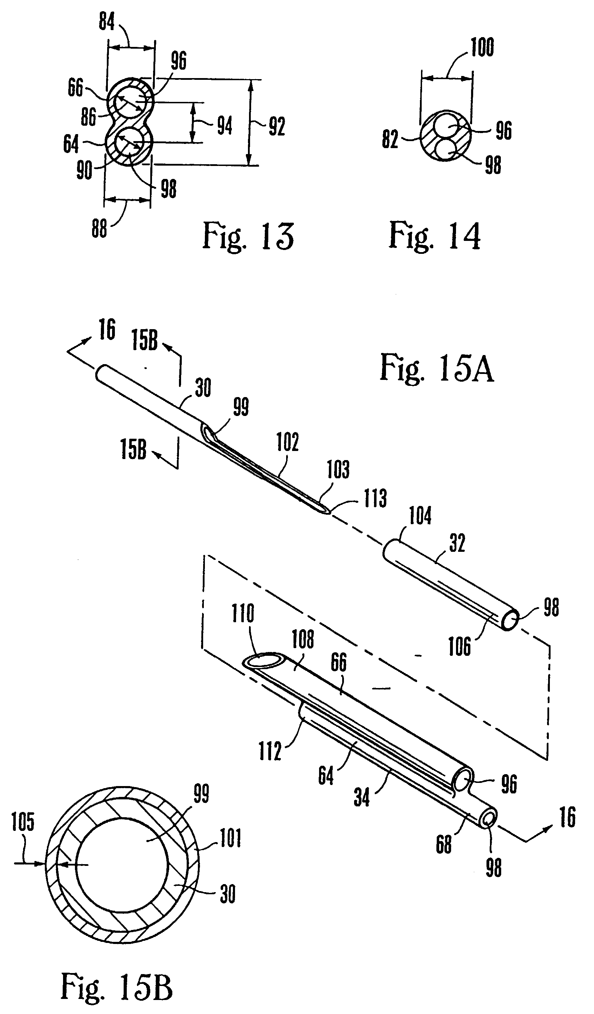

As can be appreciated by cross-referencing Fig. 12 with Figs. 13 and 14, the necked-down portion 82 of dual lumen tube 34 has a smaller, more compact, cross sectional profile (Fig. 14) than does the rest of the dual lumen tube 34 (Fig. 13). The purpose for this difference in cross sectional areas is that the necked-down portion 82 is thereby able to present a slimmer profile near the balloon assembly 26. This reduced profile is particularly desirable where the catheter 20 is most likely to confront lesions or narrower vessels in the patient's vascular system.

By way of example, as shown in Fig. 13, the guidewire section 66 of dual lumen tube 34 will have an outer diameter 84 that is equal to about 0.625 mm (twenty five thousandths of an inch) (0.025 ± 0.003), and an inner diameter 86 that is equal to about 0.475 mm (nineteen thousandths of an inch) (0.019 ± 0.005). On the other hand, the inflation section 64 of dual lumen tube 34 will have an outer diameter 88 that is equal to about 0.55 mm (twenty two thousandths of an inch) (0.022 ± 0.003), and an inner diameter 90 that is equal to about 0.375 mm (fifteen thousandths of an inch) (0.015 ± 0.001). The height 92 of the dual lumen tube 34 will be approximately 1.15 mm (forty six thousandths of an inch) (0.046 ± 0.003) and the center-to-center distance 94 between the guidewire lumen 96 and the inflation lumen 98 in dual lumen tube 34 is approximately 0.55 mm (twenty two thousandths of an inch (0.022 ± 0.002)). Contrast this with the dimensions of the necked-down portion 82 shown in Fig. 14. There it will be seen that the overall outside diameter 100 of the necked-down portion 82 will be approximately 0.85 mm (thirty four thousandths of an inch) (0.034 ± 0.003). Within this configuration, the guidewire lumen 96 will have a diameter of approximately 0.425 mm (seventeen thousandths of an inch (0.017 ± 0.005)), and the inflation lumen 98 will have a diameter of approximately 0.3 mm (twelve thousandths of an inch (0.012 ± 0.005)).

The joining of the hypotube subassembly 22 with the mid-section subassembly 24 by thermal bonding is made possible by the fact that the hypotube 30 is pre-coated with a polymer. By cross referencing Figs. 15A and 15B it will be seen and appreciated that the hypotube 30 is formed with an inflation lumen 99 and is covered over its outer surface with a coating 101. Preferably, the polymer used to pre-coat the hypotube 30 is a Pebax material such as those used for other components of the catheter 20 and is approximately greater than about 0.125 to 0.25 mm (five - ten thousandths of an inch thick (0.0005 in)). More specifically, the polymer coating is extruded onto the outer surface of the hypotube in a high speed continuous operation. For purposes of the present invention, the hypotube 30 is preferably made of stainless steel. To do this, it is to be appreciated that the hypotube 30 is straightened prior to having the polymer extruded onto its outer surface. The method whereby the hypotube subassembly 22 is manufactured for integration into the catheter 20 is noteworthy and is discussed in greater detail below. As for the configuration of the hypotube 30 used for the catheter 20 of the present invention, its structure is, perhaps, best appreciated with reference to Fig. 15A. There it will be seen that the distal end 102 of hypotube 30 has been shaved to create a skived projection 103 for this purpose. Specifically, the skived projection 103 of the distal end 102 of hypotube 30 is dimensioned for insertion into the inflation lumen 98 at the proximal end 104 of the mid-tube 32, and for subsequent heat bonding therewith. Before this is accomplished, however, the distal end 106 of mid-tube 32 will be heat bonded to the dual lumen tube 34.

In Fig. 15A it can be seen that guidewire section 66 of the dual lumen tube 34 includes a proximally oriented extension 108 which is skived or shaved to create a guidewire port 110 for the guidewire lumen 96. To be in scale with other dimensions given herein, the extension 108 will project beyond the proximal end 112 of the inflation section 64 of dual lumen 34 to a distance of about ten millimeters (10 mm). Accordingly, as the mid-tube 32 is joined with the dual lumen tube 34, the distal end 106 of mid-tube 32 will abut the proximal end 112 of inflation section 64 of the dual lumen tube 34. Also, the extension 108 of guidewire section 66 will be in contact with the outer surface of the distal end 106 of mid-tube 32.

As indicated in Fig. 16, an RF coil 114, acting in concert with an active RF mandrel (not shown), can be used to heat bond the distal end 106 of mid-tube 32 to the dual lumen tube 34. As also indicated in Fig. 16, an RF coil 116 can be similarly used to heat bond the distal end 102 of hypotube 30 to the proximal end 104 of mid-tube 32 at a point that is just proximal to the distal end 102. Importantly, with this bond, the skived projection 103 of distal end 102 extends through mid-tube 32 to a point where the extreme distal tip 113 of the hypotube 30 is slightly distal to the guidewire port 110 in order to provide some degree of stiffness for the catheter 20 in this region. Also, with this bond, the inflation lumen 99 of hypotube 30 is placed in fluid communication with the inflation lumen 98 of dual lumen tube 34.

In light of the above disclosure it is to be appreciated that the guidewire lumen 96 of catheter 20 extends distally through the catheter 20 from the guidewire port 110 in mid-section subassembly 24 to the distal tip 38 of the balloon subassembly 26. Thus, along its course, the guidewire lumen 96 passes through the guidewire section 66 of dual lumen tube 34 and through the guidewire-marker tube 40. On the other hand, the inflation lumen 98 of catheter 20 establishes fluid communication all the way from the luer fitting 28 to the balloon 36. Specifically, the inflation lumen 98 extends successively from the inflation lumen 99 of hypotube 30, through the mid-tube 32, and the inflation section 64 of dual lumen tube 34. From the dual lumen tube 34, the inflation lumen 98 empties directly through the lumen 77 of coupling tube 41 into the interior of balloon 36.

The contributions of each of the subassemblies 22, 24 and 26 to the overall functionality of the catheter 20 are noteworthy. Along the length of the catheter 20, it is the hypotube subassembly 22 which provides most of the axial strength that is necessary to give the catheter 20 good "pushability." With its different structure, the dual lumen tube 34 of mid-section subassembly 24 provides good fluid transfer properties for inflation and deflation operations of the balloon 36. At the same time, the dual lumen tube 34 allows for improved twisting and turning performance in the distal portion of the catheter 20. Specifically, due to the dual lumen design of the tube 34 there is increased resistance to potential collapsing of the inflation lumen 98 during an advancement of the catheter 20 through a patient's vascular system. The use of a coupling tube 41 provides additional separation between points on the catheter 20 which will react to an inflation of the balloon 36. This additional separation reduces the adverse consequences which can result when the catheter 20 is stretched and permanently distorted. Further, the coupling tube 41 establishes a "coaxial" arrangement between the inflation lumen 98 and the guidewire lumen 96 which benefits both the flexibility of the catheter 20 and the inflatability of the balloon 36. In the balloon subassembly 26, the marker bands 42a,b allow for good observation of the advancement of the balloon 36 and its precise position in a patient's vascular system. Further, due to the materials used in their manufacture, the marker bands 42a,b provide for enhanced flexibility of the balloon subassembly 22. Still further, due to their dimensions, the marker bands 42a,b provide a reduced and slimmer profile which allows the distal extremities of the catheter 20 to be more easily advanced farther into the patient's vascular system.

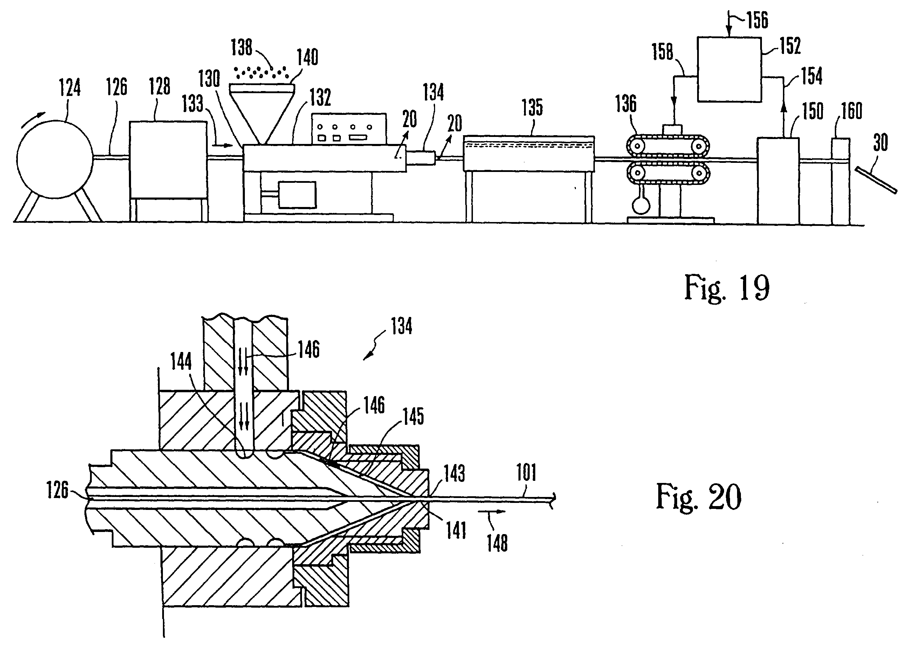

As discussed above, there must be a compatibility of materials in order for the respective hypotube, mid-section and balloon subassemblies 22, 24, 26 to be thermally bonded together. As the hypotube 30 is made of stainless steel in the preferred embodiment, in order to be bonded to the midsection subassembly 24, the hypotube 30 must be laminated with a polymer coating 101 to form the hypotube subassembly 22 of the present invention. To do this, and referring now to Fig. 19, a spool 124 of stainless steel tubing 126 is provided. The stainless steel tubing 126 is first fed into a wire straightener 128, and then into the crosshead 130 of an extruder 132. The wire straightener 128 is required because the extrusion process, wherein the polymer coating 101 is laminated onto the hypotube 30, involves very close tolerances. More specifically, and referring briefly back to Fig. 15B, the coating 101 can have a thickness 105 in a range of substantially 0.125 to 0.25 mm (five thousandths of an inch to ten thousandths of an inch). In the preferred embodiment of the invention, a four plane wire straightener 128 is used. Once straightened, the tubing 126 is fed into the crosshead 130 of the extruder 132, in a direction as shown by arrow 133.

As shown in Fig. 19, a polymer material 138 is introduced into the hopper 140 of the extruder 132. For a detailed description of the extruder 132, refer to U.S. Patent Application Serial No. 09/108,656 for an invention entitled "Medical Devices Made By Rotating Mandrel Extrusion", which is assigned to the same assignee as the present invention. The actual lamination of polymer material 138 onto the tubing 126 is, however, perhaps best appreciated by referring to Fig. 20. Fig. 20 shows the die 134 in greater detail. In Fig. 20, it is shown that the tubing 126 passes through die bore 141 and exits from the die opening 143 of extruder 132. Specifically, the polymer material 138, which is in a molten state while in the die 134, enters through feed passageway 144, and into the extrusion chamber 145, as shown by arrows 146. As the polymer material 138 is extruded through the die opening 143 at a fixed rate, the tubing 126 is pulled through the die opening 143 of extruder 132 at a variable rate in the direction shown by arrow 148. Importantly, as the tubing 126 is pulled through the die opening 143, the polymer material 138 forms around the outer surface of the tubing 126 to create the laminated coating 101 (see Fig. 15B).

Referring back to Fig. 19, after exiting the die 134, the laminated tubing 126 is cooled as it is pulled through the water bath 135 by the puller 136. Actually, the water bath 135 cools both the tubing 126 and the coating 101, and allows the coating 101 to solidify around the tubing 126. The diameter of the laminated tubing 126 is then measured by a laser micrometer 150. This diameter reading is used by a control system 152 to maintain the thickness 105 of the coating 101 in accordance with a predetermined value as chosen by the operator, as discussed below.

It is to be appreciated by referring to Fig. 19 that, to maintain the correct thickness 105 of the coating 101, a signal 156 establishing the desired thickness 105 is used as an input for the control system 152. The laser micrometer 150 measures the actual diameter of the laminated tubing 126 and generates a micrometer output signal 154, which is sent to the control system 152. The diameter of the laminated tubing 126 can be used to manipulate the thickness 105 of the coating 101 because the stainless steel tubing diameter remains constant. With a constant stainless steel tubing diameter, any changes in diameter of the laminated tubing 126 would be due to a change in thickness 105 of the coating 101. The control system 152 then compares the desired thickness signal 156 to the output micrometer signal 154 to generate an error signal. If the error signal indicates the actual laminar coating 101 is too thin, the control system 152 sends a controller output signal 158 to the puller 136 to slow the rate at which the tubing 126 is pulled through the extruder 132. As the puller 136 slows this rate at which tubing 126 is pulled, the tubing 126 will pass through the die bore 141 more slowly. Because the tubing 126 passes through the die bore 141 more slowly and the rate of extrusion of polymer 138 is constant, the tubing 126 is exposed to the molten polymer material 138 for a longer period of time, and a thicker layer of polymer material 138 forms on the tubing 126. In this manner, the thickness 105 of the laminar coating 101 can be increased.

On the other hand, if the error signal indicates the laminar coating 101 is too thick, the control system 152, in response to a signal 154 generated by the laser micrometer 150, generates a control output signal 158 which increases the pull rate on the tubing 126. Accordingly, the tubing 126 is pulled through the die bore 141 more quickly. The result then is that less polymer material 138 coats the tubing 126 and a thinner coating 101 is obtained. The result of the method of the present invention is a tubing 126 which has a substantially uniform laminar coating 101 of a desired thickness 105 (see Fig. 15B).

After the laser micrometer 150 has verified the thickness 105 of the coating 101 is at the desired value, the tubing 126 is cut by the cutter 160 to form the laminated hypotube 30 of the present invention. After being cut, a luer fitting 28 may be attached to the hypotube 30 to form the hypotube subassembly 22 of the present invention as discussed above. The hypotube subassembly 22 is then skived and assembled within the catheter 20 as also described above.

In the operation of the catheter 20 of the present invention, and as shown in Fig. 18, a guidewire 118 is positioned in the vascular system of a patient, as desired. The proximal end 120 of guidewire 118 is then inserted into the guidewire lumen 96 at the distal tip 38 of the catheter 20. The catheter 20 is then advanced along the guidewire 118 until the proximal end 120 of guidewire 118 emerges from the guidewire port 110. By grasping the proximal end 120, the guidewire 118 can be stabilized during further advancement of the catheter 20 over the guidewire 118. This advancement continues until the balloon 36 is positioned in the vascular system of the patient, as desired. With the balloon 36 so positioned, an inflator 122, which is engaged in fluid communication with the inflation lumen 98 of luer fitting 28, is activated to inflate the balloon 36. Subsequently, the balloon 36 can be deflated and the catheter 20 withdrawn over the guidewire 118. For obvious health reasons, as intended for the present invention, the catheter 20 is to be discarded after use.

## Claims

1. A radiopaque marker for locating a medical device in vivo which comprises:
a catheter body;
a matrix material; and
a metal, said metal being blended into said matrix material to form a radiopaque band, said band being bonded to said catheter body to contrast therewith.

2. A medical catheter which comprises:
a catheter body;
a radiopaque band made of a metal blended with a matrix material, said radiopaque band being thermally bonded to said catheter; and
a tubular shaped balloon having a first tail and a second tail, said first tail and said second tail being respectively thermal bonded to said catheter body to position at least a portion of said catheter body inside said balloon, with said radiopaque band being positioned on said catheter body to identify a location for said balloon on said catheter body.

3. A marker as recited in claim 1 or a catheter as in claim 2 wherein said matrix is made of a polyether block amide co-polymer.

4. A marker as recited in claim 1 or a catheter as in claim 2 wherein said catheter body is made of a polyether block amide co-polymer.

5. A marker as recited in claim 1 or a catheter as in claim 2 wherein said metal is selected from a group including tungsten, silver, gold, platinum and their alloys.

6. A marker or catheter as recited in claim 5 wherein said metal is at least seventy percent by weight of said radiopaque band.

7. A marker as recited in claim 1 or any claim dependent thereon, or a catheter as in claim 2 or any claim dependent thereon wherein said band is formed as a ring, said ring having an outer diameter and being formed with a lumen defined by an inner diameter.

8. A marker or catheter as recited in claim 7 wherein said outer diameter and said inner diameter have a difference therebetween to define a wall thickness, and said wall thickness is in the range of approximately (0.025 to 0.05 mm (one thousandth of an inch to two thousandths of an inch (0.001 -0.002 inch)).

9. A marker or catheter as recited in claim 8 wherein said catheter body is a tube and said band receives said catheter tube in said lumen to circumscribe at least a portion of said tube.

10. A marker or catheter as recited in claim 9 further comprising a tubular shaped balloon having a first tail and a second tail, said first tail and said second tail being respectively bonded to said catheter body to position at least a portion of said catheter body inside said balloon, with said radiopaque band being positioned on said catheter body to identify a location for said balloon on said catheter body.

11. A marker as recited in claim 1 or any claim dependent thereon, or a catheter as in claim 2 or any claim dependent thereon, further comprising a plurality of said radiopaque bands.

12. A method for thermally bonding a radiopaque marker to a catheter body which comprises the steps of:
blending a metal with a matrix material to form a radiopaque band;
positioning said radiopaque band against a portion of said catheter body; and
applying RF energy to said radiopaque band and to said catheter body to thermally bond said radiopaque band to said catheter body.

13. A method as recited in claim 12 wherein said band is formed as a ring, said ring having an outer diameter and being formed with a lumen defined by an inner diameter, and wherein said catheter body is a tube, and further wherein said positioning step is accomplished by inserting at least a portion of said catheter body into said lumen of said ring.

14. A method as recited in claim 13 wherein said applying step further comprises the steps of:
placing an active RF mandrel into said catheter body tube;
surrounding said radiopaque marker, said catheter body tube and said mandrel with an RF coil to locate said radiopaque marker and said catheter body tube between said RF coil and said active RF mandrel; and
sending a current through said coil.

15. A method as recited in any of claims 12 to 14 wherein said matrix material and said catheter body are made of a polyether block amide co-polymer.

16. A method as recited in any of claims 12 to 15 wherein said metal is selected from a group including tungsten, silver, gold, platinum and their alloys, and wherein said metal is at least seventy percent by weight of said radiopaque band.

17. A method as recited in any of claims 12 to 16 wherein said catheter body further comprises a tubular shaped balloon having a first tail and a second tail, and said method further comprises the step of respectively bonding said first tail and said second tail to said catheter body to position at least a portion of said catheter body inside said balloon, with said radiopaque band being positioned on said catheter body to identify a location for said balloon on said catheter body.

18. A dual lumen tube subassembly for use in a single-operator-exchange (SOE) medical balloon catheter to interconnect the balloon with an inflator and to establish a guidewire passageway for the catheter, said subassembly comprising:
a guidewire section formed with a guidewire lumen, said guidewire section having a proximal end and a distal end;
an inflation section formed with an inflation lumen, said inflation section having a proximal end and a distal end, said inflation section being longitudinally juxtaposed with said guidewire section to create a proximal extension for said guidewire section and to create a distal extension for said inflation section;
a guidewire marker tube thermally bonded to said distal extension of said inflation section and to said guidewire section to establish a continuous guidewire passageway between said guidewire marker tube and said guidewire section; and
a mid-tube section thermally bonded to said proximal extension of said guidewire section and to said inflation section to establish a fluid passageway between said inflation section and said mid-tube section for inflating said balloon.

19. A device as recited in claim 18 wherein said guidewire section is composed of a polymer material to provide a color contrast between said guidewire section and said mid-tube section.

20. A device as recited in claim 19 wherein said polymer is a medical grade plastic.

21. A device as recited in claim 20 wherein said medical grade plastic is a polyether block amide co-polymer.

22. A device as recited in claim 21 wherein said co-polymer is Pebax.

23. A device as recited in any of claims 18 to 22 wherein said mid-tube section and said inflation section and said guidewire marker tube are made of compatible materials.

24. A device as recited in any of claims 18 to 23 wherein said proximal extension of said guidewire section is approximately ten millimeters (10 mm) long.

25. A device as recited in any of claims 18 to 24 wherein said distal extension of said inflation section is approximately two millimeters (2 mm) long.

26. A method for forming a dual lumen tube for use in a single-operator-exchange (SOE) medical balloon catheter, said dual lumen tube interconnecting the balloon with an inflator and establishing a guidewire passageway for the catheter, said method comprising the steps of:
extruding said dual lumen tube, said dual lumen tube having a proximal end and a distal end and having a guidewire section and an inflation section, said guidewire section being formed with a guidewire lumen, and said inflation section being formed with an inflation lumen, said guidewire lumen being longitudinally juxtaposed with said inflation lumen;
cutting said proximal end of said dual lumen tube to create a proximal extension for said guidewire section extending longitudinally beyond said inflation section;
cutting said distal end of said dual lumen tube to create a distal extension for said inflation section extending longitudinally beyond said guidewire section;
thermally bonding a guidewire marker tube to said distal extension of said inflation section and to said guidewire section to establish a continuous guidewire passageway between said guidewire marker tube and said guidewire section; and
thermally bonding a mid-tube section to said proximal extension of said guidewire section and to said inflation section to establish a fluid passageway between said inflation section and said mid-tube section for inflating said balloon.

27. A method as recited in claim 26 wherein said dual lumen tube is made of a material of a polymer material which provides a color contrast between said guidewire section and said mid-tube section.

28. A method as recited in claim 26 to 27 further comprising the step of skiving said proximal extension of said guidewire section prior to said thermal bonding of said mid-tube section to said inflation section and to said extension of said guidewire section.

29. A method as recited in claim 26, 27 or 28 wherein said thermal bonding is accomplished using radio frequency (RF) energy.

30. A method as recited in any of claims 26 to 29 wherein said thermal bonding is accomplished at substantially 177°C (three hundred and fifty degrees Fahrenheit (350 F)).

31. A method as recited in any of claims 26 to 30 wherein said cutting of said proximal end is accomplished to make said proximal extension of said guidewire section approximately ten millimeters (10 mm) long.

32. A method as recited in any of claims 26 to 31 wherein said cutting of said distal end is accomplished to make said distal extension of said inflation section approximately two millimeters (2 mm) long.

33. A hypotube subassembly for establishing an inflation airway for a medical balloon catheter which comprises:
a hypotube having an outer surface, a proximal end, and a distal end, said distal end being shaved to form a skived projection;
a coating positioned over said outer surface of said hypotube; and
a mid-tube having a proximal end and a distal end, said mid-tube being formed with a lumen for receiving said skived projection of said hypotube therein with said proximal end of said mid-tube being bonded to said coating on said hypotube proximal to said distal end of said hypotube and said distal end of said mid-tube being connected to said balloon to establish said airway through said hypotube to said balloon.

34. A hypotube subassembly as recited in claim 33 wherein said skived projection extends substantially through said mid-tube.

35. A hypotube subassembly as recited in claim 33 or 34 wherein said coating is made of a polymer material and said mid-tube is made of a polymer material.

36. A hypotube subassembly as recited in claim 35 wherein said polymer material of said coating and said polymer material of said mid-tube are polyether block amid co-polymers.

37. A hypotube subassembly as recited in claim 36 wherein said polymer material of said coating is a Pebax 7030 and said polymer material of said mid-tube is a Pebax 7233.

38. A hypotube subassembly as recited in any of claims 33 to 37 wherein said hypotube is approximately one meter in length, said skived projection of said hypotube is approximately fifty five mm in length, and said mid-tube is approximately sixty mm in length.

39. A hypotube subassembly as recited in any of claims 33 to 38 further comprising a luer fitting connected to said proximal end of said hypotube.

40. A hypotube subassembly defining a longitudinal axis and formed with a lumen extending along said axis for establishing an inflation airway for a medical balloon catheter between an inflator and a balloon, said hypotube subassembly comprising:
a first section having a wall surrounding said lumen, said wall of said first section including a reinforcement completely surrounding said lumen to establish a longitudinal stiffness for said hypotube subassembly in said first section, said reinforcement having a skived projection extending therefrom; and
a second section extending longitudinally from said first section and having a wall surrounding both said lumen and said skived extension of said reinforcement to reduce the longitudinal stiffness in said second section, relative to said first section, and establish increased flexibility for said second section, relative to said first section.

41. A hypotube subassembly as recited in claim 40 wherein said reinforcement is a hypotube.

42. A hypotube subassembly as recited in claim 40 or 41 wherein said skived projection extends substantially through said second section.

43. A hypotube subassembly as recited in claim 40, 41 or 42 wherein said reinforcement has an outer surface wherein said wall of said first section includes a coating of polymer material positioned over said outer surface of said reinforcement, and further wherein said wall of said second section is a polymer material.

44. A hypotube subassembly as recited in claim 43 wherein said polymer material of said coating and said polymer material of said second section are polyether block amid co-polymers.

45. A hypotube subassembly as recited in claim 44 wherein said polymer material of said coating is a Pebax 7030 and said polymer material of said mid-tube is a Pebax 7233.

46. A hypotube subassembly as recited in any of claims 40 to 45 wherein said first section Is approximately one meter in length, said skived projection of said reinforcement is approximately fifty five mm in length, and said section is approximately sixty mm in length.

47. A hypotube subassembly as recited in any of claims 40 to 46 further comprising a luer fitting connected to said first section with said first section being located between said luer fitting and said second section.

48. A device for providing an air passageway to inflate a balloon of a medical balloon catheter and for providing stiffness to increase pushability of said medical balloon catheter which comprises:
a metal hypotube having a distal end, a proximal end and an outer surface;
a skived projection extending distally from said distal end of said metal hypotube;
a polymer coating attached to said outer surface;
a polymer mid-tube formed with a lumen for receiving said skived projection therein to place said hypotube in fluid communication with said mid-tube, said polymer mid-tube being thermally bonded to said coating to position and hold said skived projection in said lumen of said polymer mid-tube; and
means for connecting said polymer mid-tube in fluid communication with said balloon to inflate said balloon through said hypotube.

49. A device as recited in claim 48 wherein said skived projection extends substantially through said mid-tube, and wherein said coating and said mid-tube are made of a polymer material.

50. A device as recited in claim 49 wherein said polymer material of said coating and said polymer material of said mid-tube are polyether block amid co-polymers.

51. A device as recited in claim 50 wherein said polymer material of said coating is a Pebax 7030 and said polymer material of said mid-tube is a Pebax 7233.

52. A device as recited in any of claims 48 to 51 wherein said hypotube is approximately one meter in length, said skived projection is approximately fifty five mm in length, and said mid-tube is approximately sixty mm in length, and wherein said device further comprises a luer fitting connected to said proximal end of said hypotube.

53. An inflatable balloon subassembly for a medical catheter which comprises:
an inflatable balloon having a proximal tail and a distal tail;
a guidewire tube having a proximal end and a distal end, said distal tail of said balloon being connected to said distal end of said guidewire tube in a surrounding relationship thereto; and
a catheter body formed with a guidewire lumen and an inflation lumen, said proximal end of said guidewire tube being connected to said catheter body in fluid communication with said guidewire lumen thereof to establish a passageway for receiving a guidewire therethrough, and said proximal tail of said balloon being connected to said catheter body to establish fluid communication between said balloon and said inflation lumen of said catheter body.

54. A balloon subassembly as recited in claim 53 further comprising a coupling tube formed with a lumen and having a proximal end and a distal end, said proximal tail of said balloon being connected to said distal end of said guidewire tube in a surrounding relationship thereto, and said proximal end of said coupling tube being connected to said catheter body.

55. A balloon subassembly as recited in claim 54 wherein said balloon, said guidewire tube, said catheter body and said coupling tube are made of compatible polymers.

56. A balloon subassembly as recited in claim 54 or 55 wherein said coupling tube is approximately five centimeters in length (5 cm).

57. A balloon subassembly as recited in claim 54, 55 or 56 wherein said guidewire lumen and said inflation lumen of said catheter body are longitudinally juxtaposed.

58. A balloon subassembly as recited in any of claims 53 to 57 wherein said coupling tube and said guidewire tube are coaxial.

59. A balloon subassembly as recited in any of claims 53 to 58 wherein said balloon is thermally bonded to said guidewire tube, said guidewire tube is thermally bonded to said catheter body, and said coupling tube is thermally bonded to said balloon and thermally bonded to said catheter body.

60. An inflatable balloon subassembly for a medical catheter which comprises:
a dual lumen tube having a first lumen and a second lumen;
a single lumen tube connected in fluid communication with said first lumen of said dual lumen tube to establish a passageway for receiving a guidewire therethrough;
a coupling tube having a lumen for receiving said single lumen tube therethrough, said coupling tube being connected to said dual lumen tube in fluid communication with said second lumen thereof; and
a balloon having a distal tail and a proximal tail, said distal tail of said balloon being connected to said single lumen tube in a surrounding relationship thereto and said proximal tail of said balloon being connected to said coupling tube in fluid communication therewith.

61. A balloon subassembly as recited in claim 60 wherein said coupling tube is approximately five centimeters in length (5 cm).

62. A balloon subassembly as recited in claim 60 or 61 wherein said first lumen and said second lumen of said dual lumen tube are longitudinally juxtaposed, and wherein said coupling tube and said single lumen tube are coaxial.

63. A balloon subassembly as recited in claim 60, 61^or 62 wherein said balloon is thermally bonded to said single lumen tube, said single lumen tube is thermally bonded to said dual lumen tube, and said coupling tube is thermally bonded to said balloon and thermally bonded to said dual lumen tube.

64. A balloon subassembly as recited in any of claims 60 to 63 wherein said balloon, said single lumen tube, said dual lumen tube and said coupling tube are made of compatible polymers.

65. A balloon subassembly as recited in claim 64 wherein said polymer is Pebax.

66. A method for manufacturing an inflatable balloon subassembly for a medical catheter which comprises the steps of:
providing a tubular shaped balloon having a distal tail and a proximal tail, a guidewire tube formed with a lumen and having a distal end and a proximal end, a coupling tube formed with a lumen and having a distal end and a proximal end, and a dual lumen tube formed with a first lumen and a second lumen;
thermally bonding said distal tail of said balloon to said distal end of said guidewire tube in a surrounding relationship therewith;
thermally bonding said proximal end of said guidewire tube in fluid communication with said first lumen dual lumen tube to establish a passageway for receiving a guidewire therethrough;
thermally bonding said proximal end of said coupling tube to said dual lumen tube to surround said guidewire tube and to establish fluid communication between said lumen of said coupling tube and said second lumen of said dual lumen tube; and
thermally bonding said proximal tail of said balloon to said distal end of said coupling tube to establish fluid communication therebetween.

67. A method as recited in claim 66 wherein said balloon, said guidewire tube, said dual lumen tube and said coupling tube are made of compatible polymers.

68. A method as recited in claim 66 or 67 wherein said coupling tube is approximately five centimeters in length (5 cm).

69. A method as recited in claim 66, 67 or 68 wherein said first lumen and said second lumen of said dual lumen tube are longitudinally juxtaposed.

70. A method as recited in any of claims 66 to 69 wherein said coupling tube and said guidewire tube are coaxial.

71. A method for manufacturing a medical balloon catheter having a hypotube subassembly for use in interconnecting the balloon of the catheter with an inflator, comprising the steps of:
providing a source of metal tubing, said tubing having an outer surface;
straightening said tubing;
pulling said tubing at a variable rate through a die opening of an extruder in response to a control signal;
extruding a polymer material through said die opening to form a laminar coating of said polymer material on said outer surface of said tubing as said tubing is pulled through said die opening, said coating having a thickness;
cooling said tubing and said coating;
measuring said thickness of said coating to generate said control signal for said pulling step to maintain a substantially uniform thickness for said coating
cutting said tubing and said coating to form a laminated tube, said tube having a length with a proximal end and a distal end;
fixing a luer hub fitting to said proximal end of said tube;
engaging said inflator with said luer hub fitting to establish fluid communication between said inflator and said proximal end of said tube; and
attaching an inflatable balloon in fluid communication with said distal end of said tube.

72. A method as recited in claim 71 wherein said source of metal tubing is a spool of stainless steel tubing.

73. A method as recited in claim 71 or 72 wherein said straightening step is accomplished with a four plane wire straightener.

74. A method as recited in claim 71, 72 or 73 wherein said rate of said pulling step is approximately 15 m per minute (fifty feet per minute (50 ft/min)).

75. A method as recited in any of claims 71 to 74 wherein said laminar coating is a polymer material made of a medical grade plastic.

76. A method as recited in claim 75 wherein said medical grade plastic is a polyether block amide co-polymer.

77. A method as recited in claim 76 wherein said co-polymer is Pebax.

78. A method as recited in any of claims 71 to 78 wherein said coating is colored blue to contrast said hypotube subassembly within said catheter.

79. A method as recited in any of claims 71 to 78 wherein said measuring step is accomplished with a laser micrometer, said micrometer having a control system for generating said control signal for said pulling step.

80. A method as recited in any of claims 71 to 79 wherein said attaching step is accomplished with thermal bonding.

81. A method as recited in claim 80 wherein said thermal bonding is accomplished using radiofrequency (RF) energy at approximately 177°C (three hundred and fifty degrees Fahrenheit (350 F)).

82. A method as recited in any of claims 71 to 81, further comprising the step of:
skiving said distal end of said tube, said skiving step to be accomplished after said fixing step and before said attaching step.

83. A medical balloon catheter having a hypotube subassembly for interconnecting the balloon with an inflator, comprising:
a metal tube having an outer surface, a proximal end and a distal end;
a polymer coating laminated onto said outer surface of said tube, said coating being laminated on said outer surface by passing said tube through a die bore of an extruder and extruding a polymer material through said die bore onto said surface to obtain a substantially uniform thickness for said coating on said tube;
a luer fitting bonded to said polymer coating at said proximal end of said tube, said luer fitting being engageable with an inflator; and
an inflatable balloon subassembly bonded to said polymer coating at said distal end of said tube to place said inflator in fluid communication with said balloon.

84. A device as recited in claim 83 wherein said thickness of said coating is measured to generate a control signal and said tube is passed through said die bore at a rate responsive to said control signal.

85. A device as recited in claim 83 or 84 wherein said polymer material is a medical grade plastic and said metal tube is made of stainless steel.

86. A device as recited in claim 85 wherein said medical grade plastic is a polyether block amide co-polymer.

87. A device as recited in claim 86 wherein said co-polymer is Pebax.

88. A device as recited in any of claims 83 to 87 wherein said tube is approximately one thousand and twenty millimeters (1020 mm) in length.

89. A device as recited in any of claims 83 to 88 wherein said coating is colored blue, for the purpose of contrasting said hypotube subassembly within said catheter.

90. A distal tip for advancing a medical catheter over a guidewire which comprises:
a tubular shaped balloon having a proximal tail and a distal tail, said balloon being made of a first polymer; and
a tube for receiving the guidewire therethrough, said tube having a proximal end and a distal end, said tube being made of a second polymer, said distal end of said tube being affixed to said distal tail of said balloon to establish an integral bond therebetween.

91. A distal tip as recited in claim 90 wherein both said first polymer and said second polymer are a polyether block amide co-polymer.

92. A distal tip as recited in claim 90 or 91 wherein said first polymer and said second polymer are made of the same material.

93. A distal tip as recited in claim 90, 91 or 92 wherein both said first polymer and said second polymer are miscible with each other.

94. A distal tip as recited in claim 90, 91 or 92 wherein said distal tip is formed with an end taper and a transition taper, said transition taper being proximal to said end taper and contiguous therewith, and wherein both said end taper and said transition taper have an increasing diameter in the proximal direction:

95. A distal tip as recited in claim 94 wherein said tube defines a longitudinal axis and said end taper is characterized by an angle α which is measured from the longitudinal axis of said tube and is in the range of from about fifteen degrees to about seventy-five degrees (α = 15 - 75).

96. A distal tip as recited in claim 94 wherein said tube defines a longitudinal axis and said transition taper is characterized by an angle β which is measured from the longitudinal axis of said tube and is in the range of from about four degrees to about ten degrees (β = 4° - 10°).

97. A distal tip as recited in any of claims 90 to 96 wherein said tube is formed with a lumen for receiving a guidewire therethrough.

98. A distal tip for a medical catheter which comprises:
an end taper; and
a transition taper, said transition taper being proximal to said end taper and contiguous therewith, and wherein both said distal taper and said transition taper have an increasing diameter in the proximal direction.

99. A distal tip as recited in claim 98 wherein said medical catheter comprises:
a tubular balloon having a proximal end and a distal end with said distal end being integrally bonded to said tip; and
a tube having a proximal end and a distal end, said tube being positioned inside said balloon with said distal end of said tube integrally bonded to said balloon and to said tip.

100. A distal tip as recited in claim 99 wherein said tube defines a longitudinal axis and said end taper is characterized by an angle α which is measured from the longitudinal axis of said tube and is in the range of from about fifteen degrees to about seventy-five degrees (α = 15 - 75).

101. A distal tip as recited in claim 100 wherein said transition taper is characterized by an angle β which is measured from the longitudinal axis of said tube and is in the range of from about four degrees to about ten degrees (β = 4° - 10°).

102. A distal tip as recited in claim 99 wherein said balloon is made of a first polymer, said tube is made of a second polymer, and said tip is made of a melt combination of said first polymer and said second polymer.

103. A distal tip wherein said first polymer and said second polymer are the same polymer.

104. A method for manufacturing the distal tip of an angioplasty balloon catheter which comprises the steps of:
providing a tube having a proximal end and a distal end with a lumen formed through said tube therebetween, said tube being made of a first polymer;
inserting a mandrel into said lumen of said tube;
positioning a tubular balloon having a proximal tail and a distal tail to surround said tube and to place said distal tail of said balloon over said distal end of said tube, said tubular balloon being made of a second polymer;
introducing said distal end of said tube with said distal tail of said balloon into a cavity mold to hold said distal end of said tube and said distal tail of said balloon between said mold and said mandrel; and
energizing said mold to melt said first polymer of said distal end of said tube and said second polymer of said distal tail of said balloon to establish an integral bond therebetween.

105. A method as recited in claim 104 wherein said mandrel is an active RF mandrel.

106. A method as recited in claim 104 or 105 wherein both said first polymer and said second polymer are a polyether block amide co-polymer.

107. A method as recited in claim 104 or 105 wherein said first polymer and said second polymer are made of the same material.

108. A method as recited in claim 104 or 105 wherein both said first polymer and said second polymer are miscible with each other.

109. A method as recited in any of claims 104 to 108 wherein said energizing step forms said distal tip with an end taper and a transition taper, said transition taper being proximal to said end taper and contiguous therewith, and wherein both said distal taper and said transition taper have an increasing diameter in the proximal direction and wherein said tube defines a longitudinal axis and said end taper is characterized by an angle α which is measured from the longitudinal axis of said tube and is in the range of from about fifteen degrees to about seventy-five degrees (α = 15 - 75 ), and further wherein said transition taper is characterized by an angle β which is measured from the longitudinal axis of said tube and is in the range of from about four degrees to about ten degrees (β = 4° - 10°).

110. A medical catheter which comprises:
a hypotube subassembly including a core tube, said core tube being formed with an inflation lumen extending therethrough;
a mid-section subassembly having a proximal end and a distal end, said mid-section subassembly having a guidewire lumen longitudinally juxtaposed with an inflation lumen, said proximal end of said mid-section subassembly being thermally bonded with said hypotube subassembly to connect said inflation lumen of said core tube in fluid communication with said inflation lumen of said mid-section subassembly; and
a balloon subassembly including a balloon having a proximal tail and a distal tail and a guidewire tube having a proximal end and a distal end, said distal tail of said balloon being attached to said guidewire tube proximal said distal end of said guidewire tube, and said proximal tail of said balloon being thermally bonded to said distal end of said mid-section subassembly to interconnect said guidewire tube with said guidewire lumen of said mid-section subassembly and to connect said balloon in fluid communication with said inflation lumen of said mid-section subassembly.

111. A catheter as recited in claim 110 wherein said mid-section subassembly is made of a polymer material and said hypotube subassembly further comprises a polymer coating on said core tube for thermally bonding said mid-section subassembly to said coating of said hypotube subassembly.

112. A catheter as recited in claim 110 further comprising a coupling tube having a proximal end and a distal end, said distal end of said coupling tube being connected with said proximal tail of said balloon and said proximal end of said coupling tube being connected to said distal end of said mid-section subassembly.

113. A catheter as recited in claim 112 wherein said coupling tube is approximately five centimeters in length and is made of a polymer material for thermally bonding said coupling tube to said mid-section subassembly.

114. A catheter as recited in any of claims 110 to 113, wherein said hypotube subassembly is selected from a plurality of substantially similar hypotube subassemblies prior to being thermally bonded to said mid-section subassembly.

115. A catheter as recited in any of claims 110 to 114, wherein said mid-section subassembly is selected from a plurality of substantially similar mid-section subassemblies prior to being thermally bonded to said hypotube subassembly and prior to being thermally bonded to said balloon subassembly.

116. A catheter as recited in any of claims 110 to 115, wherein said balloon subassembly is selected from a plurality of substantially similar balloon subassemblies prior to being thermally bonded to said mid-section subassembly.

117. A catheter as recited in any of claims 110 to 116, wherein said coating of said hypotube subassembly, said mid-section subassembly and said balloon subassembly are made of compatible materials.

118. A catheter as recited in claim 111 wherein said hollow core tube is made of stainless steel and said surrounding coating is a medical grade plastic.

119. A catheter as recited in claim 118 wherein said medical grade plastic is a polyether block amide co-polymer.

120. A catheter as recited in claim 119 wherein said medical grade plastic coating is colored for the purpose of visually contrasting said hypotube sub assembly within said catheter.

121. A catheter as recited in any of claims 110 to 120 wherein said thermal bonding is accomplished by the use of radio frequency (RF) energy.

122. A catheter as recited in claim 121 wherein said thermal bonding is accomplished at approximately 177°C (350 degrees Fahrenheit (350 °F)).

123. A catheter as recited in any of claims 110 to 122 wherein said hypotube subassembly is approximately one thousand and twenty millimeters (1020 mm) in length.

124. A catheter as recited in any of claims 110 to 123 wherein said mid-section subassembly is approximately three hundred millimeters (300 mm) in length.

125. A method for manufacturing a catheter comprising the steps of:
manufacturing a plurality of hypotube subassemblies, each said hypotube subassembly including a core tube, said core tube being formed with an inflation lumen extending therethrough;
constructing a plurality of a mid-section subassemblies, each said mid-section having a guidewire lumen longitudinally juxtaposed with an inflation lumen;
fabricating a plurality of balloon subassemblies, each said balloon subassembly including a balloon having a proximal tail and a distal tail and a guidewire tube having a proximal end and a distal end and a coupling tube having a proximal end and a distal end, said distal tail of said balloon being attached to said guidewire tube proximal said distal end of said guidewire tube, and said distal end of said coupling tube being attached to said proximal end of said balloon;
selecting one hypotube subassembly from said plurality of hypotube subassemblies;
selecting one mid-section subassembly from said plurality of mid-section subassemblies;
selecting one balloon subassembly from said plurality of balloon subassemblies;
thermally bonding said hypotube subassembly to said proximal end of said selected mid-section subassembly to place said inflation lumen of said core tube in fluid communication with said inflation lumen of said mid-section subassembly; and
thermally bonding said proximal end of said coupling tube to said distal end of said mid-section subassembly to interconnect said guidewire tube with said guidewire lumen of said mid-section subassembly, and to connect said balloon in fluid communication with said inflation lumen of said mid-section subassembly.

126. A method as recited in claim 125 wherein said thermal bonding is accomplished using radio frequency (RF) energy.

127. A method as recited in claim 125 or 126, wherein said thermal bonding is accomplished at approximately 177°C (three hundred fifty degrees Fahrenheit (350° F)).
